# EUROPEAN PATENT SPECIFICATION

(11) **EP 2 961 430 B1**
(45) Date of publication and mention of the grant of the patent: **16.08.2017**
(21) Application number: 14707170.8
(22) Date of filing: 28.02.2014
(51) Int. Cl.: A61K 35/00, A61K 35/28, G01N 33/00, A61P 37/00, A61P 29/00

(54) **PROTECTION OF THE VASCULAR ENDOTHELIUM FROM IMMUNOLOGICALLY MEDIATED CYTOTOXIC REACTIONS WITH HUMAN CD34-NEGATIVE PROGENITOR CELLS**
SCHUTZ DES VASKULÄREN ENDOTHELIUMS VOR IMMUNOLOGISCH VERMITTELTEN ZYTOTOXISCHEN REAKTIONEN MIT MENSCHLICHEN CD34-NEGATIVEN VORLÄUFERZELLEN
PROTECTION DE L'ENDOTHÉLIUM VASCULAIRE CONTRE DES RÉACTIONS CYTOTOXIQUES À MÉDIATION IMMUNOLOGIQUE AVEC DES CELLULES PROGÉNITRICES CD34-NÉGATIVES HUMAINES

(30) Priority: 01.03.2013 EP 13157426; 28.03.2013 EP 13161666
(43) Date of publication of application: 06.01.2016
(73) Proprietor: Apceth GmbH & Co. KG, 81377 München (DE)
(72) Inventor: EISSNER, Günther, 81545 München (DE); GUENTHER, Christine, 81479 Munich (DE); HUSS, Ralf, 83666 Waakirchen (DE)
(74) Representative: Hertin und Partner Rechts- und Patentanwälte PartG mbB
(86) International application number: PCT/EP2014/053923
(87) International publication number: WO 2014/131877

(56) References cited:
- WO-A1-2011/041240
- WO-A1-2011/076414
- WO-A1-2012/083021
- WO-A2-2012/020308
- WO-A2-2014/011407
- US-A1- 2012 213 743
- J. TOLAR, K.; LE BLANC, A. KEATING; B. R. BLAZAR: "Concise Review: Hitting the Right Spot with Mesenchymal Stromal Cells", STEM CELLS, vol. 28, 2010, pages 1446-1455, XP002711583, cited in the application
- ANDREA L GEORGE ET AL: "Endothelial progenitor cell biology in disease and tissue regeneration", JOURNAL OF HEMATOLOGY & ONCOLOGY, vol. 4, no. 1, 1 January 2011 (2011-01-01) , pages 24-24, XP055075248, ISSN: 1756-8722, DOI: 10.1146/annurev.biochem.67.1.395

## Description

### Field of the Invention

This invention is related to human CD34-negative mesenchymal stem cells for medical use in the treatment of clinical conditions.

### Background of the Invention

Human adult CD34-negative progenitor cells are multipotent cells with the capacity for self-renewal and multilineage differentiation into various tissues of the hematopoietic, endothelial and mesenchymal variety. CD34-negative progenitor cells have been associated with immunomodulatory and regenerative potential which makes them interesting for use as a cell therapeutic agent in treating human disease.

For instance, international patent application WO 2008/150368 A1 discloses medical use of non-genetically modified CD34-negative stem cells in the treatment of gastrointestinal disorder, diabetes, muscular dystrophy, and acute wound healing in surgery or physical trauma. Singer and Caplan describes putative mechanisms of action of human mesenchymal stem cells in inflammation (N. G. Singer and A. I. Caplan: "Mesenchymal Stem Cells: Mechanisms of Inflammation", Annual Review of Pathology: Mechanisms of Disease, 2011, 457-478). Tolar et al. review the controversies and recent insights into MSC biology, the regulation of alloresponses by MSCs in preclinical models, as well as clinical experience with MSC infusion (J. Tolar, K. Le Blanc, A. Keating, B. R. Blazar: "Concise Review: Hitting the Right Spot with Mesenchymal Stromal Cells", Stem Cells 2010, 28, 1446-1455). Roemeling-van Rhijn et al. provides results from preclinical and clinical MSC research in solid organ transplantation (M. Roemeling-van Rhijn, W. Weimar, M. J. Hoogduijn: "Mesenchymal Stem Cells: Application for Solid Organ Transplantation", Current Opinion in Organ Transplantation, 2012, 17, 55-62). Likewise, Hoogduijn et al. evaluates the progression of mesenchymal stem cell therapy in clinical organ transplantation (M. J. Hoogduijn, F. C. Popp, A. Grohnert, M. J. Crop, M. van Rhijn, A. T. Rowshani, E. Eggenhofer, P. Renner, M. E. Reinders, T. J. Rabelink, L. J. van der Laan, F. J. Dor, J. N. Ijzermans, P. G. Genever, C. Lange, A. Durrbach, J. H. Houtgraaf, B. Christ, M. Seifert, M. Shagidulin, V. Donckier, R. Deans, O. Ringden, N. Perico, G. Remuzzi, A. Bartholomew, H. J. Schlitt, W. Weimar, C. C. Baan, M. H. Dahlke, and the MISOT study group: "Advancement of Mesenchymal Stem Cell Therapy in Solid Organ Transplantation (MISOT)", Transplantation, 90, 2010, 124-126). LeBlanc and co-workers investigated whether mesenchymal stem cells could ameliorate graft-versus-host-disease (GvHD) after hematopoietic stem cell transplantation (K. Le Blanc, F. Frassoni, L. Ball, F. Locatelli, H. Roelofs, I. Lewis, E. Lanino, B. Sundberg, M. E. Bernardo, M. Remberger, G. Dini, R. M. Egeler, A. Bacigalupo, W. Fibbe, O. Ringdén, Developmental Committee of the European Group for Blood and Marrow Transplantation: "Mesenchymal Stem Cells for Treatment of Steroid Resistant, severe, acute Graft-versus-Host-Disease: A Phase Two Study", Lancet, 2008, 371, 1579-86). Pati and co-workers suggested that MSCs can therapeutically target vascular permeability and inflammation through local and systemic effects in the lungs induced by hemorrhagic shock (S. Pati, M. H. Gerber, T. D. Menge, K. A. Wataha, Y. Zhao, J. A. Baumgartner, J. Zhao, P. A. Letourneau, M. P. Huby, L. A. Baer, J. R. Salsbury, R. A. Kozar, C. A. Wade, P. A. Walker, P. K. Dash, C. S. Cox Jr, M. F. Doursout, J. B. Holcomb: "Bone marrow derived mesenchymal stem cells inhibit inflammation and preserve vascular endothelial integrity in the lungs after hemorrhagic shock", PLoS One, 2011, 6,e25171). Charbord reviews the multiple characteristics of bone marrow mesenchymal stem cells including stromal and immumomodulatory capacities, which may account for the versatility of the mechanisms of injured tissue repair (P. Charbord: "Bone marrow mesenchymal stem cells: historical overview and concepts", Human Gene Therapy, 2010, 21, 1045-56).

The increasing clinical interest in CD34-negative progenitor cells is accompanied by a strong need for better understanding of their therapeutic potential and a more stratified medical use of these multifunctional cells.

### Summary of the Invention

This invention is as defined in the present claims and provides human CD34-negative mesenchymal stem cells for use as a medicament for protecting the vascular endothelium from CD8+ cytotoxic T-lymphocyte mediated cytotoxic reactions of a subject at risk of, or afflicted with, vascular inflammatory disease.

Also disclosed herein is a method for producing human CD34-negative mesenchymal stem cells for the above use, comprising the method steps of
a) isolating the CD34-negative mesenchymal stem cells,
b) expanding the CD34-negative mesenchymal stem cells for at least 12 days in a cell growth medium,
c) harvesting the CD34-negative mesenchymal stem cells.

This invention further provides a method for determining the ability of CD34-negative mesenchymal stem cells to protect the vascular endothelium from immunologically mediated cytotoxic reactions by preparing a sample comprising endothelial target cells, cytotoxic CD8+ T-lymphocytes and CD34-negative mesenchymal stem cells and a reference sample comprising endothelial target cells and cytotoxic CD8+ T-lymphocytes without CD34-negative mesenchymal stem cells, and comparing the lysis of endothelial target cells in the sample and the reference sample.

### Brief Description of the Drawings

Figure 1 is a block diagram showing a representative experimental design of the method for determining the ability of CD34-negative progenitor cells of the present invention to protect the vascular endothelium from immunologically mediated cytotoxic reactions.
Figure 2 shows the result of protection of endothelial cells from specific lysis by allogenic CD8+ cytotoxic T-lymphocytes by CD34-negative progenitor cells of the present invention using bone marrow mesenchymal stem / stromal cells from different donors.
Figure 3 shows that lysis of endothelial cells by allogenic CD8+ CTLs is MHC class I restricted and independent of natural killer cell or lymphokine-activated killer cell activity.
Figure 4 shows that protection of the endothelial cells from lysis by allogenic CD8+ cytotoxic T-lymphocytes is specific to bone marrow MSC (BM-MSC), whereas size-matched control cells do not exhibit a protective effect.
Figure 5 shows the result of the comparison of the level of endothelial protection by CD34-negative progenitor cells of the present invention using mesenchymal stem / stromal cells derived from various tissues.

### Terms and Definitions

In this application, certain terms are used which all have the meanings set forth as follows.

As used herein, a cell is "allogenic" with respect to the subject if it, or any of its precursor cells, is from another subject of the same species.

As used herein, "CD34-negative progenitor cell" shall mean a stem cell lacking CD34 on its surface. CD34-negative progenitor cells as such can also give rise or differentiate into CD34-negative stem /stromal cells. CD34-negative progenitor cells can comprise hematopoietic, endothelial and/or mesenchymal progeny. In certain preferred embodiments, "CD34-negative progenitor cell" shall mean a CD34-negative mesenchymal stem / stromal cell (MSC); while hematopoietic and also endothelial progenitor cells eventually start to express CD34 and other concurrent markers during maturation.

As used herein, "vascular endothelium" shall include, with limitation, cells that line the interior surface of blood vessels. In particular, the vascular endothelium comprises endothelial cells in direct contact with blood.

As used herein, "immunologically mediated cytotoxic reactions" shall mean, without limitation, the cell mediated immune response that leads to damage or death of the target cell at which the immune response is directed. In certain embodiments, immunologically mediated cytotoxic reactions shall include MHC -mediated cellular immunity.

As used herein, "CD34-negative mesenchymal stem / stromal cell" shall mean a stem cell fulfilling the three minimal criteria proposed by the International Society for Cellular Therapy: (1) plastic-adherence when maintained in standard culture conditions using tissue culture flasks; (2) expression of CD105, CD73 and CD90, as measured by flow cytometry, and lack of expression of CD45, CD34, CD14 or CD11b, CD79a or CD19; (3) ability to differentiate into osteoblasts, adipocytes and chondroblasts under standard *in vitro* differentiation conditions (M. Dominici, K. Le Blanc, I. Mueller, I. Slaper-Cortenbach, F. Marini, D. Krause, R. Deans, A. Keating, Dj. Prockop, E. Horwitz: "Minimal criteria for defining multipotent mesenchymal stromal cells. The International Society for Cellular Therapy position statement", Cytotherapy, 2006, 8, 315-7).

In certain embodiments, "CD34-negative mesenchymal stem / stromal cell" shall additionally mean an MSC expressing B7-H1 (PD-L1) after stimulation with gamma-IFN (M. Najar, G. Raicevic, H.F. Kazan, C. De Bruyn, D. Bron, M. Toungouz, L. Lagneaux: "Immune-related antigens, surface molecules and regulatory factors in human-derived mesenchymal stromal cells: the expression and impact of inflammatory priming", Stem Cell Rev. 2012, 8, 1188-98); S. Tipnis, C. Viswanathan, A.S. Majumdar: "Immunosuppressive properties of human umbilical cord-derived mesenchymal stem cells: role of B7-H1 and IDO", Immunol Cell Biol., 2010, 88, 795-806; P. Fiorina, M. Jurewicz, A. Augello, A. Vergani, S. Dada, S. La Rosa, M. Selig, J. Godwin, K. Law, C. Placidi, R.N.Smith, C. Capella, S. Rodig, C.N. Adra, M. Atkinson, M.H. Sayegh, R. Abdi: "Immunomodulatory function of bone marrow-derived mesenchymal stem cells in experimental autoimmune type 1 diabetes", J Immunol. 2009, 183, 993-1004; C.J. Chang, M.L. Yen, Y.C. Chen, C.C. Chien, H.I. Huang, C.H. Bai, B.L. Yen: "Placenta-derived multipotent cells exhibit immunosuppressive properties that are enhanced in the presence of interferon-gamma", Stem Cells, 2006, 24, 2466-77).

Unless further specified, "vascular inflammatory disease" shall mean, without limitation, an immune response triggering inflammation of vascular tissue, including large and small arteries, veins and lymphatics.

As used herein, "subject" shall mean any animal, such as human, non-human primate, mouse, rat, guinea pig or rabbit. Preferably, the subject is human.

As used herein, "protecting the vascular endothelium" shall mean slowing, stopping or reversing the progression of the immunologically mediated cytotoxic reactions towards any vascular tissue.

### Description of the Invention

The vascular endothelium is the primary target in a variety of vascular inflammatory disorders. The inventors of the present invention realised that the specific protection of the endothelium in terms of a risk-adapted, individualized prophylaxis and/or therapeutic intervention would be of great clinical and health economical value.

The inventors undertook elaborate research of the activation state and the vitality of the vascular endothelium and discovered that CD34-negative mesenchymal stem cells are able to suppress endothelium-specific cytotoxic reactions in a pharmacological use and dose-dependent manner.

Therefore, in a first aspect, this invention provides human CD34-negative mesenchymal stem cells for use as a medicament for protecting the vascular endothelium from CD8+ cytotoxic T-lymphocyte mediated cytotoxic reactions of a subject at risk of, or afflicted with, vascular inflammatory disease.

In one embodiment of the invention, the vascular inflammatory disease comprises vascular endothelium specific cell lysis caused by CD8+ cytotoxic T-lymphocytes. The inventors elucidated that the endothelium was a direct target for CD8+ cytotoxic T-lymphocytes in certain conditions of vascular inflammatory disease.

The therapeutic use of CD34-negative mesenchymal stem cells was particularly effective in interfering with CD8+ cytotoxic T-lymphocyte mediated cytotoxic reactions.

In a specific embodiment of the invention, the CD8+ cytotoxic T-lymphocytes comprise endothelium-specific cytotoxic T lymphocytes which are CD27-negative and CD28-negative. The inventors surprisingly found evidence for the existence of endothelium-specific cytotoxic T lymphocytes which are CD27-negative and CD28-negative and do not recognize hematopoietic targets. These cells exhibit phenotypically and functionally remarkable characteristics. For instance, their lytic activity is enhanced by CD4+/CD25+/FoxP3+ regulatory T-lymphocytes (TRegs). The inventors accomplished significant inhibition of endothelial cell lysis by this particular type of CTLs when CD34-negative mesenchymal stem cells were administered.

This invention therefore provides a stratified therapeutic use of CD34-negative mesenchymal stem cells, enabling a risk-adapted, individualized prophylaxis and/or therapy with CD34-negative mesenchymal stem cells. Correspondingly, advantageous medical use of CD34-negative mesenchymal stem cells according to the present invention includes the clinical conditions defined below.

In one embodiment of this invention, the vascular inflammatory disease comprises an alloreaction against the vascular endothelium of a solid organ transplant which is allogenic with respect to the subject, and/or acute, inflammatory or allergic vasculitis as a result of an auto-immune response and/or chronic inflammation of the vascular endothelium.

In another embodiment, the vascular inflammatory disease comprises transplant-related complications after allogenic hematopoietic stem cell transplantation, wherein the transplant-related complications comprise preferably graft-versus-host-disease (GvHD) and/or microangiopathic disease.

For example, the transplant-related complications comprise graft-versus-host-disease (GvHD). In particular, the graft-versus-host-disease can be characterized by predominantly selective damage of the gastrointestinal tract, the liver, the skin including the mucosa, the pulmonary system, and combinations thereof. In certain embodiments of the invention, the graft-versus-host-disease comprises steroid-refractory acute or chronic GvHD.

Another example of transplant-related complications is microangiopathic disease, such as, for instance, hepatic veno-occlusive disease (VOD).

The human CD34-negative mesenchymal cells can also be used for protecting the vascular endothelium in vascular inflammatory disease comprising acute, inflammatory or allergic vasculitis as a result of an auto-immune response. This use includes, without limitation, allergic granulomatosis, giant cell arteritis, Wegener granulomatosis, Takayasu arteritis, Kawasaki disease, Thromangitis obliterans (Buerger disease), polyarteritis nodosa, Churg-Strauss-Syndrome, microscopic polyangitis, cryoglobulinemic vasculitis, urticarial vasculitis, Behcet disease, Goodpasture syndrome, post-infectious vasculitis and drug-induced vasculitis.

In another embodiment, the vascular inflammatory disease comprises chronic inflammation of the vascular endothelium. Chronic inflammation can, for example, comprise atherosclerosis. Chronic inflammation can also comprise vasculitis associated with rheumatoid disease.

According to the invention, the CD34-negative progenitor cells comprise CD34-negative mesenchymal stem / stromal cells. The CD34-negative mesenchymal stem cells can be selected, for instance, from a group comprising bone marrow, umbilical cord, placenta and adipose tissue CD34-negative mesenchymal stem cells, and combinations thereof. In a preferred embodiment of the invention, the CD34-negative mesenchymal stem cells are bone marrow CD34-negative mesenchymal stem cells.

Preferably, the CD34-negative mesenchymal stem cells have the ability to reduce the immunologically mediated cytotoxic reactions against vascular endothelial cells by at least 50% compared to endothelial cells without protection by CD34-negative mesenchymal cells. A suitable method for determination of the reduction of immunologically mediated cytotoxic reactions according to the present invention is detailed further below.

The human CD34-negative mesenchymal stem cells are preferably, but not exclusively, adapted for injection into the subject's bloodstream, for instance by introducing such cells into one of the subject's veins or arteries via injection. Such administering can also be performed, for example, once or a plurality of times and/or over one or more extended periods. A single injection is preferred, but repeated injections over time may be necessary in some instances.

Preferably, the CD34-negative mesenchymal stem cells are admixed with a pharmaceutically acceptable carrier. Pharmaceutically acceptable carriers are well-known to those skilled in the art and include, but are not limited to, 0.01 to 0.1 molar and preferably 0.05 molar phosphate buffer or 0.8% saline. Moreover, such pharmaceutically acceptable carriers can be aqueous or non-aqueous solutions, suspensions, and emulsions, examples of non-aqueous solvents are propylene, glycol, polyethylene glycol, vegetable oils such as olive oil and injectable organic esters such as ethyl oleate. Aqueous carriers include water, alcoholic/aqueous solutions, emulsions and suspensions including saline and buffer media. Parenteral vehicles include sodium fluoride solution, ringer's dextrose, dextrose and sodium chloride, lactated ringers and fixed oils. Intravenous vehicles include fluid and nutrient replenishers, electrolyte replenishers such as ringers dextrose, those based on ringer's dextrose and the like. Fluids used commonly for intravenous administration are found, for example, in Remington: The Science and Practice of Pharmacy, 20th edition, page 808, Lippincott, Williams and Wilkins (2000).

Different administration regimes can be used. Preferably, the human CD34-negative mesenchymal stem cells are adapted for injection into the subject's bloodstream in a therapeutically effective amount. A therapeutically effective amount can, for instance, comprise a range of 1x10² to about 1x10⁸ cells per kilogram body weight, from about 1x10³ to about 10x10⁷ cells per kilogram body weight, from about 1x10⁴ to about 1x10⁶ cells per kilogram body weight, from about 1x10⁴ to about 1x10⁵ cells per kilogram body weight, from about 1x10⁵ to about 1x10⁶ cells per kilogram body weight, from about 5x10⁴ to about 0.5x10⁵ cells per kilogram body weight, from about 1x10³ cells per kilogram body weight, about 1x10⁴ cells per kilogram body weight, about 5x10⁴ cells per kilogram body weight, about 1x10⁵ cells per kilogram body weight, about 5x10⁵ cells per kilogram body weight, about 1x10⁶ cells per kilogram body weight and about 1x10⁷ cells per kilogram body weight. These numbers have been found to be therapeutically particularly effective in the transplantation of CD34-negative mesenchymal stem cells.

In a variant of the invention, the CD34-negative mesenchymal stem cells are used preventively for protecting the vascular endothelium of a subject at risk of vascular inflammatory disease. As used herein, the preventive use shall include, without limitation, administration of the cells to a subject who is about to receive a solid organ transplant which is allogenic with respect to the subject. In another example, the preventive use comprises administering CD34-negative mesenchymal stem cells to a subject who is about to receive an allogenic hematopoietic stem cell transplant. A further example of preventive use comprises administering CD34-negative mesenchymal stem cells to a subject who has received an allogenic transplant but has not yet developed endothelial complications. A risk of vascular inflammatory disease can, for example, also comprise a genetic disorder, an autoimmune disease, cancer, cigarette smoking, alcoholism, and combinations thereof. The inventors realized that the targeted protection of the vascular endothelium in the sense of a risk adapted, individualized prophylaxis, is of great clinical and health economical value.

Such a risk stratified use may for instance comprise, without limitation, that a patient at risk of developing endothelial complications post transplant will receive CD34-negative mesenchymal stem cells prophylactically, i.e. in advance. For example, CD34-negative mesenchymal stem cells may be administered to a subject immediately prior to, during and/or or up to about six weeks before and/or after an intervention, for instance an allogenic cell- or organ transplantation. Administration of CD34-negative mesenchymal stem cells may also expand over the acute stage and/or chronic stage of endothelial complications up to about 100 days after an intervention. In addition, CD34-negative mesenchymal stem cells may be administered therapeutically to a subject upon developing or having currently developed endothelial complications and/or symptoms characteristic thereof.

The CD34-negative mesenchymal stem cells can be allogenic with respect to the subject. The CD34-negative mesenchymal stem cells can also be autologous with respect to the subject. Alternatively, a combination of allogenic and autologous CD34-negative mesenchymal stem cells can be used.

In one example, the CD34-negative mesenchymal stem cells are autologous with respect to the solid organ transplant and allogenic with respect to the subject. In another example, the CD34-negative mesenchymal stem cells are allogenic with respect to the hematopoietic stem cell transplant and autologous with respect to the subject. Also included are cases wherein the the CD34-negative mesenchymal stem cells are allogenic with respect to the subject and the solid organ transplant or hematopoietic stem cell transplant.

In one embodiment, the CD34-negative mesenchymal stem cells are used in combination with at least one further active component. For example, the at least one further active component can have a pharmacological activity selected from the group comprising anti-inflammatory activity, anti-ischemic activity, anti-thrombotic activity, and combinations thereof. In addition or alternatively, the at least one further active component can have the ability to prevent endothelial cells from allorecognition and/or lysis. In certain examples, the at least one further active component comprises a desoxyribonucleic acid derivative, for instance Defibrotide.

Also disclosed herein is a method for producing human CD34-negative mesenchymal stem cells for any of the uses above, comprising the methods step of a) isolating the CD34-negative mesenchymal stem cells, b) expanding the CD34-negative mesenchymal stem cells for at least 12 days in a cell growth medium, c) harvesting the CD34-negative mesenchymal stem cells.

The CD34-negative mesenchymal stem cells of the invention can be isolated from tissue of the group comprising bone marrow, umbilical cord, placenta and adipose tissue, or combinations thereof in method step a). The inventors found that mesenchymal stem cells from these tissue sources are particularly suitable for protecting the vascular endothelium in vascular inflammatory disease. Preferably, the CD34-negative mesenchymal stem cells are CD34-negative mesenchymal stem / stromal cells. Most preferred are CD34-negative mesenchymal stem / stromal cells from the bone marrow.

As disclosed herein, the cell growth medium of method step b) includes a medium comprising
- a human platelet lysate free of solid matter greater than 0.22 µm in diameter, wherein the lysate constitutes from 2% to 15% of the total volume of the cell growth medium,
- a human fresh frozen plasma (FFP) filtrate free of solid matter greater than 0.22 µm in diameter, wherein the FFP filtrate constitutes from 1% to 10% of the total volume of the cell growth medium,
- heparin at a concentration of from 0 U/ml to 10 U/ml of the cell growth medium,
- L-glutamine at a concentration of from 0.5 mM to 10 mM, and
- a serum-free, low glucose medium suitable for mammalian cell growth, wherein the serum-free, low glucose medium constitutes from 75% to 97% of the total volume of the cell growth medium.

Herein, human fresh frozen plasma refers to the liquid portion of human blood that has been centrifuged, separated and frozen solid at -18°C or colder within hours of collection. The inventors accomplished expansion of CD34-negative mesenchymal stem cells in this medium which are particularly potent in protecting the vascular endothelium from immunologically mediated cytotoxic reactions. In the following, this medium will be referred to as "Bio-1" medium.

As disclosed herein, CD34-negative mesenchymal stem cells are harvested in method step c) which are predominantly adherently growing on a surface in contact with a cell culture medium, such as the walls of a cell culture dish or cell culture container.

Preferably, the method steps a) through c) are performed under good manufacturing practice (GMP) and/or current good manufacturing practice (cGMP).

With these methods, the inventors accomplished *in vitro* expansion and positive selection of better defined, highly transplantable cells with particularly high vascular endothelium protecting potency, whereas conventional methods typically yield a heterogeneous mixture of distinct subpopulations within the CD34-negative progenitor cells.

In another aspect, this invention provides a method for determining the ability of CD34-negative mesenchymal stem cells to protect the vascular endothelium from immunologically mediated cytotoxic reactions by preparing a sample comprising endothelial target cells, cytotoxic CD8+ T-lymphocytes and CD34-negative mesenchymal stem cells and a reference sample comprising endothelial target cells and cytotoxic CD8+ T-lymphocytes without CD34-negative mesenchymal stem cells, and comparing the lysis of endothelial target cells in the sample and the reference sample.

In one embodiment, the endothelial target cells and/or the CD34-negative mesenchymal stem cells are allogenic with respect to the CD8+ T-lymphocytes.

In another embodiment, the CD8+ T-lymphocytes are co-cultivated with allogenic endothelial cells in the presence of Interleukin-2 (IL-2) prior to preparing the sample and the reference sample with endothelial target cells and said CD8+ T-lymphocytes. The co-cultivation can, for instance, be maintained for at least one day, preferably for at least 3 days, more preferred for at least 5 days, and most preferred for at least 7 days.

In another embodiment, at least one further active component is added to the sample and/or the reference sample. For example, the at least one further active component can have a pharmacological activity selected from the group comprising anti-inflammatory activity, anti-ischemic activity, anti-thrombotic activity, and combinations thereof. In addition or alternatively, the at least one further active component can have the ability to prevent endothelial cells from allorecognition and/or lysis. In certain examples, the at least one further active component comprises a desoxyribonucleic acid derivative, for instance Defibrotide.

With this method, the inventors accomplished a potency assay for assessing the capacity of CD34-negative mesenchymal stem cells from different sources in protecting the vascular endothelium. Moreover, an *in vitro* test for positive prediction of the therapeutic effect of CD34-negative mesenchymal stem cells *in vivo* is realised.

The method can also be used to analyse the subject's blood for the presence and/or pathophysiological activity of endothelial-cytotoxic CD8+ T-lymphocytes prior to, during and/or after receiving a transplant and/or developing endothelial complications.

For example, in a case of a solid organ transplant which is allogenic with respect to the subject, the endothelial target cells can comprise cells derived from the solid organ donor. In another case of a solid organ transplant which is allogenic with respect to the subject, the CD34-negative mesenchymal stem cells can comprise cells derived from the solid organ donor. In certain cases of a solid organ transplant which is allogenic with respect to the subject, both the endothelial target cells and the CD34-negative mesenchymal stem cells can comprise cells derived from the solid organ donor. In addition or alternatively, the CD34-negative mesenchymal stem cells can also comprise cells which are derived from at least one third party donor which is not the subject or the solid organ donor.

In a case of allogenic hematopoietic stem cell transplantation, the endothelial target cells can comprise cells derived from the subject, i.e. the transplant recipient. In another case of allogenic hematopoietic stem cell transplantation, the CD34-negative mesenchymal stem cells can comprise cells derived from the transplant recipient. In certain cases of allogenic hematopoietic stem cell transplantation, both the endothelial target cells and the CD34-negative mesenchymal stem cells can comprise cells derived from the transplant recipient. In addition or alternatively, the CD34-negative mesenchymal stem cells can also comprise cells which are derived from at least one third party donor which is not the transplant recipient or the hematopoietic stem cell donor.

In this way, the method can not only be used for determining a subject's susceptibility to develop immunologically mediated cytotoxic reactions on an individual basis using cytotoxic CD8+ T-lymphocytes derived from the subject's blood in combination with the above-identified, case-specific endothelial target cells, but also to select CD34-negative mesenchymal stem cells which exhibit particularly potent protection of the endothelial target cells.

As a result, patients can, for instance, be stratified according to their risk of vascular inflammatory disease. Moreover, an individualised prophylaxis and/or therapy can be devised.

### Detailed Description of Embodiments

In the following examples, certain embodiments of the invention will be explained in more detail with reference to figures and experimental data. The examples and figures are not intended to be limiting with respect to specific details.

### EXAMPLE 1: Experimental design cytotoxicity assay

The following example describes an experimental setup designed by the inventors for assessing the potency of CD34-negative progenitor cells of the invention with respect to protecting the vascular endothelium from immunologically mediated cytotoxic reactions.

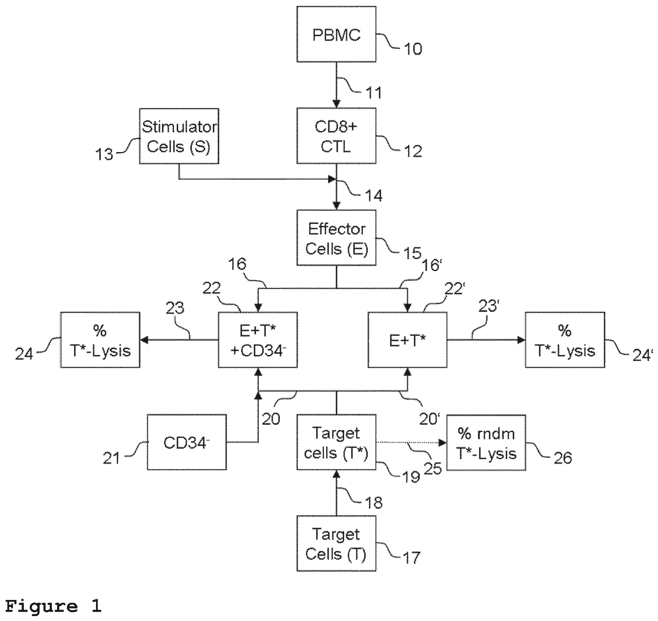

Figure 1 is a block diagram illustrating one representative embodiment of the cytotoxicity assay of the present invention. Mononuclear cells of the peripheral blood (PBMC, 10) may be used as a source of cytoptoxic cells. The PBMC can, for instance, be derived from a healthy third party donor. Alternatively, PBMC of the subject to be treated can be used.

Next, the PBMC can be further selected for CD8+ T-lymphocytes (11). The selection for CD8+ T-lymphocytes can, for instance, comprise enrichment of CD8+ T-lymphocytes from the PBMC by immunoseparation. The immunoseparation can, for instance, comprise negative selection of CD8+ T-lymphocytes by removing non-CD8+ T-lymphocytes. A suitable method for enrichment of CD8+ T-lymphocytes is, for instance, the untouched selection via immunomagnetic mircoparticles. Unwanted cells can, for instance, be targeted for removal with antibody complexes recognising CD4, CD14, CD16, CD19, CD20, CD36, CD56, CD66B, CD123, TCRγ/δ, glycophorin A and dextran-coated magnetic particles. Afterwards, the phenotypic purity of the selected cells can be verified, for instance by flow cytometry. Preferably, the majority of the selected cells are CD8+ cytotoxic T-lymphocytes (CTL, 12).

In the next phase, a co-cultivation (14) of the CD8+ CTL with endothelial stimulator cells (13) which are allogenic with respect to the CD8+ CTL can be maintained. Preferably, the endothelial stimulator cells are incapable of performing mitosis. A suitable cell line is, for instance, the SV40 large T antigen transformed microvascular endothelial cell line CDC/EU.HMEC-1 (HMEC). The co-cultivation of the endothelial stimulator cells and the CD8+ CTL can, for instance, be maintained for about seven days. Preferably, the co-cultivation further comprises stimulating the CD8+ CTL with Interleukin-2.

A preparation of endothelial target cells (17) can be subjected to a labelling step (18) in which they are labelled with a first label rendering endothelial target cells distinguishable from other non-target cells. For example, such a label can comprise a fluorescent compound having different emission characteristics when present in the plasma membrane of a cell than when outside of the plasma membrane. A suitable compound is, for instance, 3, 3' dioctadecyloxacarbocyanine perchlorate (DIOC18₃). Target cells can be, for instance, endothelial cells from transplant tissue. Target cells can be an endothelial cell line, for instance the microvascular endothelial line CDC/EU.HMEC-1.

In the next phase, a sample (22) is prepared by combining a first portion of the labelled target cells (20), a first portion of the effector cells (16), and CD34-negative progenitor cells (21) of the present invention. The CD34-negative progenitor cells of the invention can, for instance, be allogenic with respect to the endothelial target cells and the effector cells. The ratio of endothelial target cells and CD34-negative progenitor cells can, for instance, be 5:1. The ratio of effector cells to endothelial target cells can, for instance, be 20:1, 10:1 or 5:1. A reference sample (22') is prepared by combining a second portion of the labelled target cells (20') and a second portion of the effector cells (16') without CD34-negative progenitor cells, in the same ratio as in the sample.

Incubation of the sample and reference sample is maintained (23, 23'). Incubation can, for instance, be maintained for about four hours.

Afterwards, the amount of lysed endothelial target cells in the sample and the reference sample is determined (24, 24'). To this end, lysed endothelial target cells can be labelled with a second label rendering lysed target cells distinguishable from non-lysed target cells. Such a second label may, for instance, be a stain suitable for staining dead cells positively. For example, a fluorochromatic stain such as propidium iodide can be used. A suitable method for determining the amount of lysed endothelial target cells can, for instance, be flow cytometry. For example, the percentage of lysed cells can be determined by determining the amount of cells carrying both the first and second label, e.g. DIOC18₃ and PI, within the total population of cells carrying the first label, e.g. DIOC18₃, by flow cytometry.

In addition, the percentage of target cells specifically lysed by the effector cells can be corrected by subtracting the percentage of randomly lysed cells. To this end, a third portion (25) of the labelled target cells (19) is prepared which contains neither effector cells nor CD34-negative progenitor cells. The third portion is further treated (25) in the same way as the sample and the reference sample, after which the amount of randomly lysed endothelial target cells in the third portion (26) is determined as described above.

### EXAMPLE 2: Third party CD34-negative progenitor cells of the present invention protect endothelial cells from lysis by allogenic CD8+ CTL

The cytotoxicity assay described in example 1 was used to assess the lysis of endothelial target cells (HMEC) by allogenic cytotoxic T-lymphocytes with and without third party derived CD34-negative progenitor cells of the present invention. In this case, CD34-negative mesenchymal stem / stromal cells isolated from the bone marrow were used. The BM-MSCs were expanded in Bio-1 medium which resulted in favourable cell populations for therapeutic use in terms of maintaining stem cell characteristics, cell viability and potency in protecting the vascular endothelium from immunologically mediated cytotoxic reactions.

Different ratios of effector cells and target cells of 20:1, 10:1 and 5:1 were prepared. For each ratio, six individual samples without bone marrow MSC and six individual samples with bone marrow MSC (BM-MSC) were prepared. In the latter, the target cells were incubated with the BM-MSC of a single donor for 24 hours before addition of the effector cells.

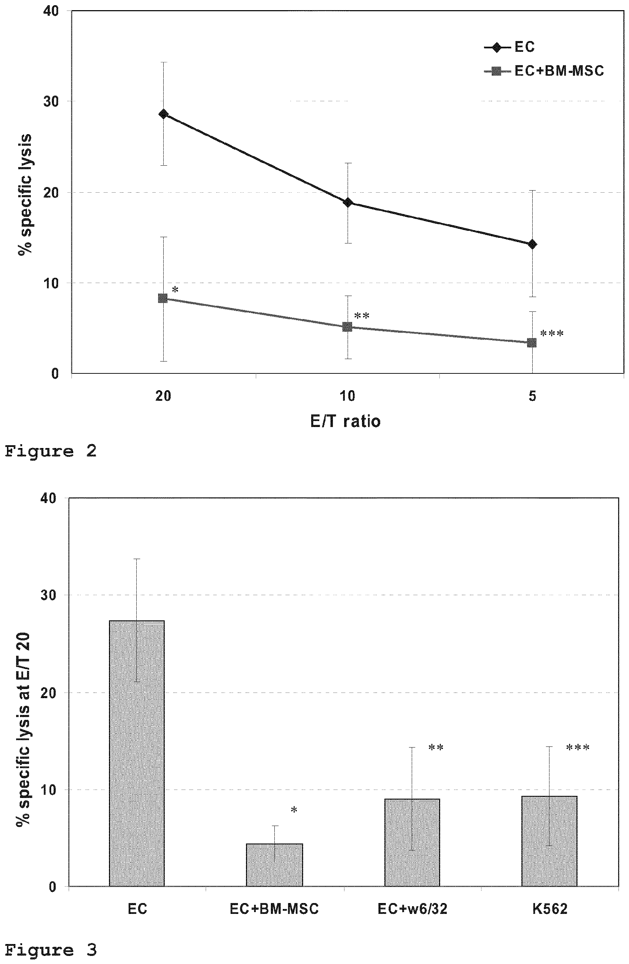

The results of the experiments are shown in Figure 2. The graph shows the specific lysis of target cells in percent over the respective effector-to-target cell ratio for samples without the BM-MSC (diamond-shaped markers) and with the BM-MSC (square markers). Data represent mean values and standard deviation of six individual experiments. It was found that the BM-MSC inhibit the lysis of allogenic endothelial cells by CD8+ CTLs with high significance in all tested E/T ratios (*, **, ***: p < 0.001). On average, the specific lysis of allogenic endothelial target cells was reduced by approximately 65%, i.e. from 28.3 ± 5.8% to 9.7 ± 8.3%, by the addition of CD34-negative bone marrow mesenchymal stem / stromal cells.

### EXAMPLE 3: Immunomodulatory action of CD34-negative progenitor cells of the present invention is endothelium-specific

As a control, an experiment was performed as described in example 2, with the difference that the CD34-negative BM-MSC were not added to the endothelial cells but pre-incubated with the effector cells and afterwards removed. In this case, no reduction in specific lysis of the endothelial target cells was observed when the effector cells were pre-incubated with the BM-MSC in comparison to effector cells that were not pre-incubated with BM-MSC. It can therefore be concluded that the immunomodulatory activity of the BM-MSC is related to a specific interaction of the BM-MSC with the endothelial target cells.

### EXAMPLE 4: Endothelial target cell lysis by allogenic CD8+ CTL is MHC class I restricted and independent of natural killer cell or lymphokine activated killer cell activity

As a further control, it was investigated whether the lytic activity of the allogenic CD8+ cytotoxic T-lymphocyte effector cells is antigen-specific by being restricted to the MHC class I presentation of the alloantigens. To this end, endothelial target cells were subjected to CD8+ effector cells as in example 2, but in the presence of a neutralizing MHC class I antibody (W6/32). In addition, it was to be shown that the effector cells do not possess an activity which corresponds to that of natural killer cells or unspecific lymphokine activated killer cells. This control was accomplished by performing an experiment as in example 2, but wherein the endothelial target cells were substituted by a control target cell line for natural killer cells (K562).

The results of these control experiments are summarized in Figure 3. The columns represent the arithmetic mean and standard deviation of the specific lysis of target cells in an effector/target ratio of 20 of four independent experiments, each with four individual BM-MSC donors. The results show that the presence of a neutralizing anti-MHC class I antibody significantly reduces the specific lysis of target cells (*, **: p < 0.001), confirming that the lytic activity of the effector cells is alloantigen specific. Furthermore, lysis of the control targets K562 was significantly reduced (***: p < 0.002), demonstrating that the cytotoxic T-lymphocyte effector cells do not possess an activity corresponding to that of natural killer cells or unspecific lymphokine activated killer cells.

### EXAMPLE 5: Protection of endothelial target cells from lysis by CD8+ CTL effector cells is specific to CD34-negative progenitor cells of the present invention

CD34-negative progenitor cells of the present invention such as bone marrow-derived CD34-negative mesenchymal stem / stromal cells are relatively large cells. Therefore, it was to be assessed whether the protection of the endothelial target cells by BM-MSC was based on a steric inhibition of the CD8+ T-lymphocyte effector cells from accessing the endothelial cells by the BM-MSC rather than on the immunomodulatory capacity of the BM-MSC. The experimental design was analogous to example 2, but wherein fibroblast-like cells from human heart tissue was added to the endothelial target cells instead of BM-MSC. The fibroblast-like cells were matched in size with BM-MSC.

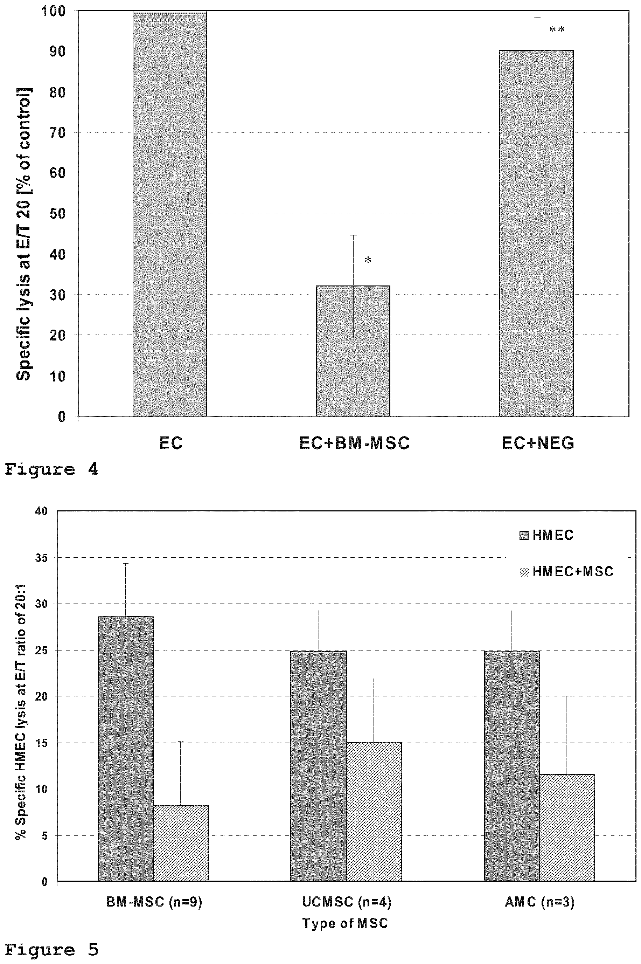

The results are shown in Figure 4. The columns represent the arithmetic mean and standard deviation of specific lysis of endothelial target cells, normalized in percent to the lysis of endothelial cells without protection, of three independent experiments each with three different BM-MSC donors. No protective effect for the endothelial target cells was observed from addition of the size-matched control cells (**: no significance), demonstrating that the protection of the endothelial target cells by BM-MSC is immunologically related and not due to steric inhibition.

### EXAMPLE 6: The CD34-negative stem cells of the present invention are immunologically naïve

It is important that the CD34-negative progenitor cells of the present invention are themselves not immunogenic. For this reason the immunogenicity of bone marrow CD34-negative mesenchymal stem / stromal cells was investigated. The experiment was performed as per example 2, but wherein not endothelial cells but bone marrow mesenchymal stem / stromal cells were used as target cells in the absence of endothelial cells. No lytic activity of the allogenic CD8+ cytotoxic T-lymphocytes effector cells was observed when BM-MSC were presented as targets. This finding is in agreement with the immunologic naivety of CD34-negative progenitor cells and mesenchymal stem / stromal cells in particular.

### Example 7: Endothelial protection capacity of CD34- progenitor cells of the present invention from different sources

The effectivity of CD34-negative progenitor cells of the present invention derived from different tissue sources in protection of endothelial target cells from CD8+ CTL-mediated lysis was compared. Experiments were performed as in Example 2, wherein the CD34⁻ progenitor cells (21) were either bone marrow-derived MSCs (BM-MSC, 9 individual samples), umbilical cord-derived MSCs (UC-MSC, 4 individual samples), or amnion membrane-derived MSCs (AMC-MSC, 3 individual samples). The results of these experiments are summarised in Figure 5. Each column pair shows the arithmetic mean and standard deviation of the specific endothelial target cell lysis in per cent without MSC protection (solid columns) and with MSC protection (hatched columns) in dependence of the MSC source. All tested MSCs were able to protect the endothelial target cells from CD8+ CTL-mediated lysis. The most potent protection was observed with BM-MSCs, where the endothelial cell lysis was reduced on average by 72.3% compared to the control without MSC protection. The AMC-MSCs reduced the endothelial cell lysis on average by approximately 53.2%, and the UC-MSCs still reduced the endothelial cell lysis on average by approximately 39.5%. This example also highlights the importance of the present method for determining the ability of CD34-negative progenitor cells of the present invention to protect the vascular endothelium from immunologically mediated cytotoxic reactions in order to assess the protection potency of cells from different sources, and to positively predict the effect of the CD34-negative progenitor cells of the present invention *in vivo.*

## Claims

1. Human CD34-negative mesenchymal stem cells for use in treating or protecting the vascular endothelium from CD8+ cytotoxic T-lymphocyte mediated cytotoxic reactions of a subject at risk of, or afflicted with, vascular inflammatory disease.

2. The human CD34-negative mesenchymal stem cells for use in the medical treatment according to the preceding claim, wherein the treatment comprises the administration of CD34-negative progenitor cells and said CD34-negative progenitor cells consist of CD34-negative mesenchymal stem cells.

3. The human CD34-negative mesenchymal stem cells for use according to the preceding claim, wherein the CD34-negative mesenchymal stem cells are administered as an admixture comprising a pharmaceutically acceptable carrier, wherein said admixture comprises CD34-negative progenitor cells and said CD34-negative progenitor cells consist of CD34-negative mesenchymal stem cells.

4. Human CD34-negative mesenchymal stem cells for use according to any of the preceding claims for protecting the vascular endothelium from CD8+ cytotoxic T-lymphocyte mediated cytotoxic reactions for prevention of vascular inflammatory disease in a subject at risk of developing post-transplant endothelial complications.

5. The human CD34-negative mesenchymal stem cells for use according to any of the preceding claims, wherein the CD8+ cytotoxic T-lymphocytes comprise endothelium-specific cytotoxic T lymphocytes which are CD27- and CD28-negative.

6. The human CD34-negative mesenchymal stem cells for use according to any of the preceding claims, wherein the vascular inflammatory disease comprises an alloreaction against the vascular endothelium of a solid organ transplant which is allogenic with respect to the subject, and/or acute, inflammatory or allergic vasculitis as a result of an auto-immune response and/or chronic inflammation of the vascular endothelium.

7. The human CD34-negative mesenchymal stem cells for use according to any of the claims 1-5, wherein the vascular inflammatory disease comprises transplant-related complications after allogenic hematopoietic stem cell transplantation, wherein the transplant-related complications comprise preferably graft-versus-host-disease (GvHD) and/or microangiopathic disease.

8. The human CD34-negative mesenchymal stem cells for use according to the previous claim, wherein the acute, inflammatory or allergic vasculitis comprises allergic granulomatosis, giant cell arteritis, Wegener granulomatosis, Takayasu arteritis, Kawasaki disease, Thromangitis obliterans (Buerger disease), polyarteritis nodosa, Churg-Strauss-Syndrome, microscopic polyangitis, cryoglobulinemic vasculitis, urticarial vasculitis, Behcet disease, Goodpasture syndrome, post-infectious vasculitis or drug-induced vasculitis.

9. The human CD34-negative mesenchymal stem cells for use according to any of the preceding claims, wherein the CD34-negative mesenchymal stem cells are selected from a group comprising bone marrow, umbilical cord, placenta and adipose tissue CD34-negative mesenchymal stem cells, or combinations thereof.

10. The human CD34-negative mesenchymal stem cells for use according to any of the preceding claims, wherein the CD34-negative mesenchymal stem cells are **characterized by** expression of CD105, CD73 and CD90, and lack of expression of CD45, CD34, CD14 or CD11 b, CD79a or CD19.

11. The human CD34-negative mesenchymal stem cells for use according to any of the preceding claims, wherein the CD34-negative mesenchymal stem cells are used in combination with at least one further pharmacologically active component.

12. The human CD34-negative mesenchymal stem cells for use according to the previous claim, wherein the at least one further pharmacological active component has a pharmacological activity selected from the group comprising anti-inflammatory activity, anti-ischemic activity, anti-thrombotic activity, and combinations thereof.

13. An in vitro method for determining the ability of CD34-negative mesenchymal stem cells to protect the vascular endothelium from immunologically mediated cytotoxic reactions from subjects at risk of or afflicted with vascular inflammatory disease in a sample comprising endothelial target cells, cytotoxic CD8+ T-lymphocytes and CD34-negative mesenchymal stem cells and a reference sample comprising endothelial target cells and cytotoxic CD8+ T-lymphocytes without CD34-negative mesenchymal stem cells, and comparing the lysis of endothelial target cells in the sample and the reference sample.

14. The method of claim 13 wherein the CD8+ T-lymphocytes are co-cultivated with allogenic endothelial cells in the presence of Interleukin-2 prior to preparing the sample and the reference sample with endothelial target cells and said CD8+ T-lymphocytes.

15. The method of claim 13 or 14 wherein at least one further pharmacologically active component is added to the sample and/or the reference sample.

## Patentansprüche

1. Menschliche CD34-negative mesenchymale Stammzellen zur Verwendung beim Behandeln oder Schützen des vaskulären Endothels vor zytotoxischen Reaktionen, die durch zytotoxische CD8+-T-Lymphozyten vermittelt werden, eines von einer entzündlichen Gefäßerkrankung bedrohten oder betroffenen Patienten.

2. Menschliche CD34-negative mesenchymale Stammzellen zur Verwendung bei der medizinischen Behandlung nach dem vorhergehenden Anspruch, wobei die Behandlung die Verabreichung von CD34-negativen Vorläuferzellen umfasst und die CD34-negativen Vorläuferzellen aus CD34-negativen mesenchymalen Stammzellen bestehen.

3. Menschliche CD34-negative mesenchymale Stammzellen zur Verwendung nach dem vorhergehenden Anspruch, wobei die CD34-negativen mesenchymalen Stammzellen als Mischung verabreicht werden, die einen pharmazeutisch annehmbaren Träger umfasst, wobei die Mischung CD34-negative Vorläuferzellen umfasst und die CD34-negativen Vorläuferzellen aus CD34-negativen mesenchymalen Stammzellen bestehen.

4. Menschliche CD34-negative mesenchymale Stammzellen zur Verwendung nach einem der vorhergehenden Ansprüche zum Schützen des vaskulären Endothels vor zytotoxischen Reaktionen, die durch zytotoxische CD8+-T-Lymphozyten-vermittelt werden, zur Prävention einer entzündlichen Gefäßerkrankung bei einem Subjekt, das von einer Entwicklung von Endothel-Komplikationen nach einer Transplantation bedroht ist.

5. Menschliche CD34-negative mesenchymale Stammzellen zur Verwendung nach einem der vorhergehenden Ansprüche, wobei die zytotoxischen CD8+-T-Lymphozyten endothelspezifische zytotoxische T-Lymphozyten umfassen, die CD27- und CD28-negativ sind.

6. Menschliche CD34-negative mesenchymale Stammzellen zur Verwendung nach einem der vorhergehenden Ansprüche, wobei die entzündliche Gefäßerkrankung eine Alloreaktion gegen das vaskuläre Endothel eines festen Organtransplantats, das allogen in Bezug auf das Subjekt ist, und/oder akute, entzündliche oder allergische Vaskulitis als Folge einer Autoimmunantwort und/oder einer chronischen Entzündung des vaskulären Endothels umfasst.

7. Menschliche CD34-negative mesenchymale Stammzellen zur Verwendung nach einem der Ansprüche 1-5, wobei die entzündliche Gefäßerkrankung transplantatbedingte Komplikationen nach allogener hämatopoetischer Stammzelltransplantation umfasst, wobei die transplantatbedingten Komplikationen vorzugsweise Graft-versus-Host-Krankheit (GvHD) und/oder Mikroangiopathie umfassen.

8. Menschliche CD34-negative mesenchymale Stammzellen zur Verwendung nach dem vorhergehenden Anspruch, wobei die akute, entzündliche oder allergische Vaskulitis allergische Granulomatose, Riesenzellarteriitis, Wegener-Granulomatose, Takayasu-Arteriitis, Kawasaki-Krankheit, Thrombangiitis obliterans (Buerger-Krankheit), Polyarteriitis nodosa, Churg-Strauss-Syndrom, mikroskopische Polyangiitis, kryoglobulinämische Vaskulitis, Urtikaria-Vaskulitis, Behcet-Krankheit, Goodpasture-Syndrom, post-infektiöse Vaskulitis oder medikamentöse Vaskulitis umfasst.

9. Menschliche CD34-negative mesenchymale Stammzellen zur Verwendung nach einem der vorhergehenden Ansprüche, wobei die CD34-negativen mesenchymalen Stammzellen aus einer Gruppe ausgewählt sind, die CD34-negative mesenchymale Stammzellen aus Knochenmark, Nabelschnur, Plazenta und Fettgewebe oder Kombinationen davon umfasst.

10. Menschliche CD34-negative mesenchymale Stammzellen zur Verwendung nach einem der vorhergehenden Ansprüche, wobei die CD34-negativen mesenchymalen Stammzellen durch eine Expression von CD105, CD73 und CD90 und einem Fehlen der Expression von CD45, CD34, CD14 oder CD11b, CD79a oder CD19 gekennzeichnet sind.

11. Menschliche CD34-negative mesenchymale Stammzellen zur Verwendung nach einem der vorhergehenden Ansprüche, wobei die CD34-negativen mesenchymalen Stammzellen in Kombination mit mindestens einer weiteren pharmakologisch aktiven Komponente verwendet werden.

12. Menschliche CD34-negative mesenchymale Stammzellen zur Verwendung nach dem vorhergehenden Anspruch, wobei die mindestens eine weitere pharmakologisch aktive Komponente eine pharmakologische Aktivität aufweist, die aus der Gruppe ausgewählt ist, die aus entzündungshemmender Aktivität, antiischämischer Aktivität, anti-thrombotischer Aktivität und Kombinationen davon besteht.

13. In-vitro-Verfahren zum Bestimmen der Fähigkeit von CD34-negativen mesenchymalen Stammzellen, das vaskuläre Endothel vor immunologisch vermittelten zytotoxischen Reaktionen bei einem Subjekt zu schützen, das von einer entzündlichen Gefäßerkrankung bedroht oder betroffen ist, in einer Probe, die endotheliale Zielzellen, zytotoxische CD8+-T-Lymphozyten und CD34-negative mesenchymale Stammzellen umfasst und einer Referenzprobe, die endotheliale Zielzellen und zytotoxische CD8+-T-Lymphozyten ohne CD34-negative mesenchymale Stammzellen umfasst, und Vergleichen der Lyse von endothelialen Zielzellen in der Probe und der Referenzprobe.

14. Verfahren nach Anspruch 13, wobei die CD8+-T-Lymphozyten zusammen mit allogenen Endothelzellen in Gegenwart von Interleukin-2 vor dem Zubereiten der Probe und der Referenzprobe mit endothelialen Zielzellen und den CD8+-T-Lymphozyten kultiviert werden.

15. Verfahren nach Anspruch 13 oder 14, wobei der Probe und/oder der Referenzprobe mindestens ein weiterer pharmakologisch aktiver Bestandteil zugesetzt wird.

## Revendications

1. Cellules souches mésenchymateuses CD34-négatives humaines pour une utilisation dans le traitement ou la protection de l'endothélium vasculaire contre des réactions cytotoxiques médiées par des lymphocytes T cytotoxiques CD8+ d'un sujet à risque de, ou touché par, une maladie vasculaire inflammatoire.

2. Cellules souches mésenchymateuses CD34-négatives humaines pour une utilisation dans le traitement médical selon la revendication précédente, dans lesquelles le traitement comprend l'administration de cellules progénitrices CD34-négatives et lesdites cellules progénitrices CD34-négatives sont constituées de cellules souches mésenchymateuses CD34-négatives.

3. Cellules souches mésenchymateuses CD34-négatives humaines pour une utilisation selon la revendication précédente, dans lesquelles les cellules souches mésenchymateuses CD34-négatives sont administrées sous la forme d'un mélange comprenant un support pharmaceutiquement acceptable, dans lesquelles ledit mélange comprend des cellules progénitrices CD34-négatives et lesdites cellules progénitrices CD34-négatives sont constituées de cellules souches mésenchymateuses CD34-négatives.

4. Cellules souches mésenchymateuses CD34-négatives humaines pour une utilisation selon l'une quelconque des revendications précédentes pour la protection de l'endothélium vasculaire contre des réactions cytotoxiques médiées par des lymphocytes T cytotoxiques CD8+ pour la prévention d'une maladie vasculaire inflammatoire chez un sujet à risque de développer des complications endothéliales à la suite d'une transplantation.

5. Cellules souches mésenchymateuses CD34-négatives humaines pour une utilisation selon l'une quelconque des revendications précédentes, dans lesquelles les lymphocytes T cytotoxiques CD8+ comprennent des lymphocytes T cytotoxiques spécifiques de l'endothélium qui sont CD27- et CD28-négatifs.

6. Cellules souches mésenchymateuses CD34-négatives humaines pour une utilisation selon l'une quelconque des revendications précédentes, dans lesquelles la maladie vasculaire inflammatoire comprend une alloréaction contre l'endothélium vasculaire d'une transplantation d'organe solide qui est allogénique par rapport au sujet, et/ou une vascularite aiguë, inflammatoire ou allergique comme résultat d'une réponse auto-immune et/ou d'une inflammation chronique de l'endothélium vasculaire.

7. Cellules souches mésenchymateuses CD34-négatives humaines pour une utilisation selon l'une quelconque des revendications 1 à 5, dans lesquelles la maladie vasculaire inflammatoire comprend des complications associées à une transplantation après une transplantation de cellules souches hématopoïétiques allogéniques, dans lesquelles les complications associées à une transplantation comprennent de préférence la maladie du greffon contre l'hôte (MGCH) et/ou une maladie microangiopathique.

8. Cellules souches mésenchymateuses CD34-négatives humaines selon la revendication précédente, dans lesquelles la vascularite aiguë, inflammatoire ou allergique comprend la granulomatose allergique, l'artérite à cellules géantes, la granulomatose de Wegener, l'artérite de Takayasu, la maladie de Kawasaki, la thromboangéite oblitérante (maladie de Buerger), la polyartérite noueuse, le syndrome de Churg-Strauss, la polyangéite microscopique, la vascularite cryoglobulinémique, la vascularite urticarienne, la maladie de Behçet, le syndrome de Goodpasture, la vascularite post-infectieuse ou la vascularite induite par des médicaments.

9. Cellules souches mésenchymateuses CD34-négatives humaines pour une utilisation selon l'une quelconque des revendications précédentes, dans lesquelles les cellules souches mésenchymateuses CD34-négatives sont choisies dans un groupe comprenant des cellules souches mésenchymateuses CD34-négatives de la moelle osseuse, du cordon ombilical, du placenta et du tissu adipeux, ou leurs combinaisons.

10. Cellules souches mésenchymateuses CD34-négatives humaines pour une utilisation selon l'une quelconque des revendications précédentes, dans lesquelles les cellules souches mésenchymateuses CD34-négatives sont **caractérisées par** l'expression de CD105, CD73 et CD90, et le manque d'expression de CD45, CD34, CD14 ou CD11b, CD79a ou CD19.

11. Cellules souches mésenchymateuses CD34-négatives humaines pour une utilisation selon l'une quelconque des revendications précédentes, dans lesquelles les cellules souches mésenchymateuses CD34-négatives sont utilisées en combinaison avec au moins un autre composant pharmacologiquement actif.

12. Cellules souches mésenchymateuses CD34-négatives humaines pour une utilisation selon la revendication précédente, dans lesquelles l'au moins un autre composant pharmacologique actif possède une activité pharmacologique choisie dans le groupe comprenant une activité anti-inflammatoire, une activité anti-ischémique, une activité antithrombotique, et leurs combinaisons.

13. Procédé *in vitro* de détermination de la capacité de cellules souches mésenchymateuses CD34-négatives à protéger l'endothélium vasculaire contre des réactions cytotoxiques à médiation immunologique chez des sujets à risque de, ou touchés par, une maladie vasculaire inflammatoire dans un échantillon comprenant des cellules endothéliales cibles, des lymphocytes T cytotoxiques CD8+ et des cellules souches mésenchymateuses CD34-négatives et un échantillon de référence comprenant des cellules endothéliales cibles et des lymphocytes T cytotoxiques CD8+ sans cellules souches mésenchymateuse CD34-négatives, et la comparaison de la lyse des cellules endothéliales cibles dans l'échantillon et l'échantillon de référence.

14. Procédé selon la revendication 13, dans lequel les lymphocytes T CD8+ sont co-cultivés avec des cellules endothéliales allogéniques en présence d'interleukine 2 avant la préparation de l'échantillon et de l'échantillon de référence avec des cellules endothéliales cibles et lesdits lymphocytes T CD8+.

15. Procédé selon la revendication 13 ou 14, dans lequel au moins un autre composant pharmacologiquement actif est ajouté à l'échantillon et/ou l'échantillon de référence.
